Europäisches Patentamt

European Patent Office

Office européen des brevets

(19)

(11) Publication number: 0 312 957
A2

## (12) EUROPEAN PATENT APPLICATION

(21) Application number: 88117240.7

(22) Date of filing: 17.10.88

(51) Int. Cl.4: A61B 17/16 , A61F 2/46

(30) Priority: 23.10.87 IT 498487 U

(43) Date of publication of application:
26.04.89 Bulletin 89/17

(84) Designated Contracting States:
AT BE CH DE ES FR GB GR LI LU NL SE

(71) Applicant: CITIEFFE S.r.l.
Via F.lli Rosselli 3
I-40012 Lippo di Calderara di Reno
(Bologna)(IT)

(72) Inventor: Mingozzi,Franco
Via Giovanni XXIII,1
I-40012 Lippo Di Calderara Di Reno(IT)

(74) Representative: Modiano, Guido et al
MODIANO, JOSIF, PISANTY & STAUB
Modiano & Associati Via Meravigli, 16
I-20123 Milan(IT)

(54) Device for removing cement from the femoral medullary canal particularly in the replacement of hip prostheses.

(57) The device (1) includes a deformable rod element (9) screwed to a handle (2) which has a rigid rod (6) screwed to its other end. The rod element (9) supports a head (11) insertable in the medullary canal for expelling a cement mass. A bush (7) is slideable on the rigid rod (6) and can abut against the handle (2).

Fig. 1

# DEVICE FOR REMOVING CEMENT FROM THE FEMORAL MEDULLARY CANAL PARTICULARLY IN THE REPLACEMENT OF HIP PROSTHESES

The present invention relates to a device for removing cement from the femoral medullary canal particularly in the replacement of hip prostheses.

As is known, hip prostheses are fixed in the medullary canal with the aid of special cementing substances.

It is however often necessary, due to various mechanical or pathological reasons, to remove the applied prosthesis and restore the cementation.

In order to ensure a firm coupling of the new prosthesis it is furthermore necessary to remove the previously existing cement substance.

This operation is currently performed proximally, sometimes empirically with equipment not designed for the specific purpose, with the result that the removal operation becomes long and troublesome and the medullary canal is not cleaned satisfactorily.

The technical aim of the present invention is to provide a device which overcomes the disadvantages of conventional methods and allows to remove the cement more completely and rapidly than the methods currently in use.

Within this aim, an object of the invention is to provide a device which can be used distally through a hole on the femoral condyle.

This aim and this object are achieved by a device for removing cement from the femoral medullary canal particularly in the replacement of hip prostheses, characterized in that it comprises a deformable rod element provided, at one end, with a handle and, at the opposite end, with a head for expelling a cement mass, said head being distally insertable in the medullary canal, a rigid rod being associated with said handle coaxially to said rod element, a bush being slideable on said being rod, said bush being capable of abutting against said handle.

Further characteristics and advantages of the invention will become apparent from the following description of a preferred embodiment, illustrated only by way of non-limitative example in the accompanying drawings, wherein:

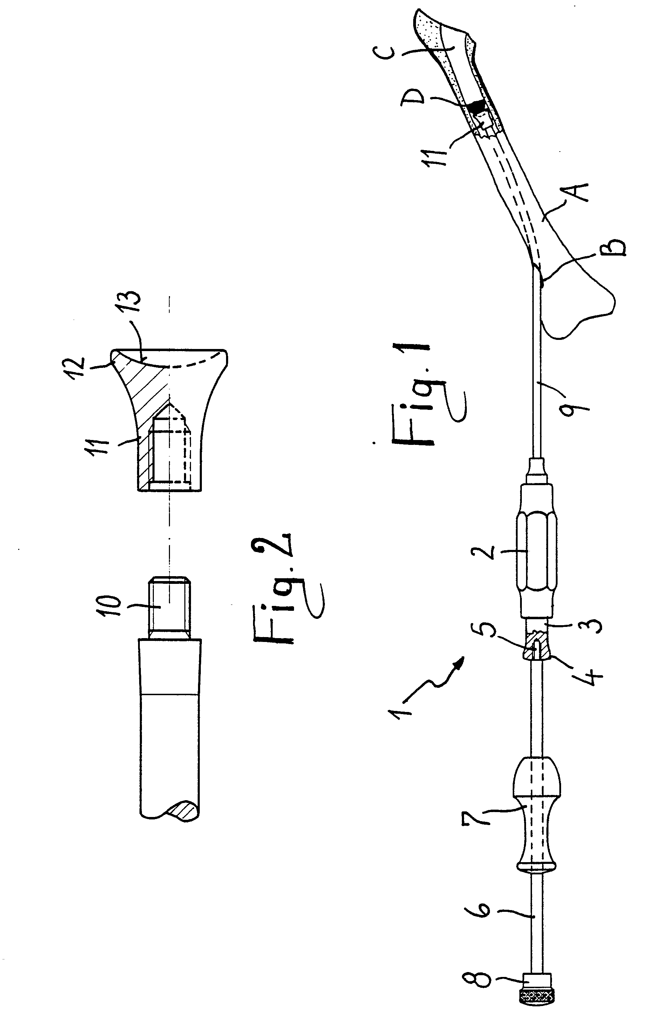

figure 1 is a view of the device in operating position and

figure 2 is a view of the detail of the head.

With reference to the above described figures, the reference numeral 1 generally indicates the device which comprises a handle 2 having a cylindrical portion 3 on one side. Portion 3 has a widened end 4 which is axially traversed by a threaded blind hole in which the threaded tang 5 of a rod 6 is screwed. A sleeve or bush 7 is guided on the rod 6 and is slideable between the portion 4 and a knob 8 fixed to the free end of the rod 6 and acting as stroke limit for the sleeve 7.

Coaxially to said rod 6, a further rod 9, having a terminal portion constituted by a threaded stem 10, is fixed to the handle 2 on the opposite side with respect to the one to which the rod 6 is applied.

A body 11 having a substantially conical shape is rigidly associated to the stem 10 and it widens in a circular end 12 which is frontally provided with a cavity 13. The body 11 constitutes the head for expelling the cement substance.

The use of the device is the following.

On the distal side with respect to the side of application of the hip prosthesis in the femur A, a hole B is drilled converging towards the medullary canal C from which it is desired to extract a cement mass D after removing the prosthesis.

The head 11 is then inserted in said hole B until it abuts against the cement mass D. The rod 9 is conveniently progressively shaped according to the path of the canal to facilitate the penetration of the head in the medullary canal.

When the head abuts against the mass D, the sleeve 7 is manually caused to slide on the rod 6 so as to repeatedly strike the end 4 of the handle 2 with an appropriately measured force. The blows on the handle are transmitted by the rod 9 to the head 11 which thus pushes the cement mass out of the canal.

Advantageously, the concave shape of the front surface of the head ensures the complete collection of any fragments which form during the blows. The risk of iatrogenic fractures of the femur is furthermore substantially reduced.

As can be seen, the invention fully achieves the intended aim and object. Furthermore the device can be equipped with a tool adapted to be inserted in the femoral canal through the proximal end to counter-act on the cement mass if required. Said tool can be constituted by a rod fully similar to the rod 9 and provided at one end with a knob and, at the opposite end, with a coupling for a head 11.

In the practical embodiment of the invention, the shapes and dimensions as well as the materials may be any according to the requirements.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Device for removing cement from the femoral medullary canal particularly in the replacement of hip prostheses, characterized in that it comprises a deformable rod element (9) provided, at one end, with a handle (2) and, at the opposite end, with a head (11) for expelling a cement mass, said head being distally insertable in the medullary canal, a rigid rod (6) being associated with said handle (2) coaxially to said rod element (9), a bush (7) being slideable on said rod (6), said bush (7) being capable of abutting against said handle (2).

2. Device according to claim 1, characterized in that said head (11) has a widened and frontally concave end (12).

3. Device according to one or more of the preceding claims, characterized in that said handle (2) has an enlarged portion, on the side of said abutting bush (7).

4. Device according to one or more of the preceding claims, characterized in that said rod element (9) and said ridig rod (6) are coaxially screwed to said handle (2).

Fig.2

Fig.1

EP 0 312 957 A2